# EUROPEAN PATENT APPLICATION

(11) **EP 2 926 848 A1**
(43) Date of publication of application: **07.10.2015**
(21) Application number: 13859241.5
(22) Date of filing: 27.11.2013
(51) Int. Cl.: A61M 5/158

(54) **SAFETY PROTECTING INFUSION NEEDLE**

(30) Priority: 27.11.2012 CN 201220635944 U
(71) Applicant: Sunwell Biotech Co., Ltd., Jiangsu (CN)
(72) Inventor: LI, Zhi-Yun, Nantong Jiangsu (CN)
(74) Representative: Baldwin, Mark
(86) International application number: PCT/CN2013/087928
(87) International publication number: WO 2014/082576

(57) **Abstract**

The present invention provides a transfusion needle with security, comprising a main body and a safety sheath. The main body is provided at the side thereof with a needle wing, and connected at one end thereof with a needle head. The front-end side and the rear-end side of the safety sheath are provided with an inclined opening and a depressed opening, respectively. The needle head is protruded out of the safety sheath when the needle wing located within the inclined opening before the transfusion needle is used, while the needle head is covered within the safety sheath when the needle wing pulled back to the depressed opening from the inclined opening after the transfusion needle has been used. The needle head may be retrieved into the safety sheath after the transfusion needle has been used, so as to avoid the medical personnel stabbed by the used needle head.

## Description

### FIELD OF THE INVENTION

The present invention relates to a transfusion needle with security, particularly to a transfusion needle with security for preventing needle prick, capable of avoiding the risk of accidental pricking wound to medical personnel caused by a needle head.

### BACKGROUND

Currently, vein transfusion needles are often used by medical personnel to apply medical treatment, such as medicament injection, blood drawing and etc. to patients. During the medical procedure, however, accidental stab wound to medical personnel is frequently incurred due to inserting and pulling out a needle head carelessly.

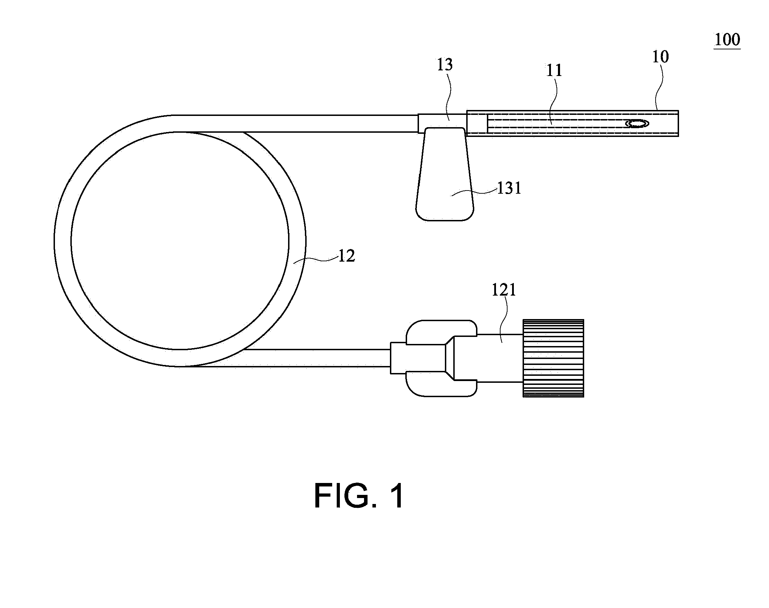

Referring to Fig. 1, there is shown a structural perspective view of an existing transfusion needle. As illustrated in the figure, the transfusion needle 100 comprises a protective needle cap 10, a needle head 11, a main body 13 and a transfusion tube 12.

Before the transfusion needle 100 is used, the needle head 11 is covered by the protective needle cap 10. The transfusion tube 12 is used as a passage for medicament injection or blood flow, and is joined at one end thereof to the needle head 11 through the main body 13, while provided at the other end thereof with a joint portion 121. The joint portion 121 is used for fitting with a pipe, conduit or vessel capable of containing medicament or blood. In addition, the main body 13 is further provided at the side thereof with a needle wing 131.

When the transfusion needle 100 is used, the protective needle cap 10 may be removed by medical personnel to expose the needle head 11 to the outside. Subsequently, the needle wing 131 is held by the medical personnel, so as to allow the needle head 11 inserting into the vein of the patient for conducting medicament injection or blood drawing.

After the transfusion needle 100 has been used, the used needle head 11 may be pulled out of the body of the patient, and the needle head 11 is then covered by the protective needle cap 10 again by the medical personnel. This protective needle cap 10 is not easy to cover precisely at once owing to its extremely small bore, most likely resulting in accidental pricking wound to the medical personnel caused by the needle head 11 due to careless manipulation, and further leading to the possibility of viral infection (such as, HIV, hepatitis, SARS and etc.). Thereby, it is very dangerous to the life safety of medical personnel.

Here, the present invention provides a transfusion needle with security for preventing needle prick, in which the enhancement of safety in retrieving needle head and the prevention of viral infection resulted from accidental stab wound to medical personnel caused by the needle head will be the objects intended to be achieved by this invention.

### SUMMARY OF THE INVENTION

It is one object of the present invention to provide a transfusion needle with security, in which a safety sheath is provided, such that an used needle head may be retrieved into the safety sheath only if a needle wing of the transfusion needle is pulled back to a rear end of the safety sheath when retrieving the needle head is intended by medical personnel, so as to avoid the medical personnel accidental stabbed by the used needle head.

It is one object of the present invention to provide a transfusion needle with security, in which the safety sheath is provided at a front side thereof with an inclined opening, and provided at a rear side thereof with a depressed opening. The needle wing is provided within the inclined opening when the transfusion needle is used, allowing the needle head entering into the vein of human body with a certain angle due to the incline-type design.

It is one object of the present invention to provide a transfusion needle with security, in which the needle wing is pulled back into the depressed opening from the inclined opening after the transfusion needle has been used, allowing the needle wing being locked within the depressed opening firmly, so as to achieve the object of retrieving the needle head safely.

For achieving the aforementioned objects, the present invention provides a transfusion needle with security, comprising: a main body, provided at the side thereof with a needle wing, and connected at one end thereof with a needle head, while connected at the other end thereof with a transfusion tube; and a safety sheath, presented as a hollow covering body for accommodating the main body, and provided at the side of a front end thereof with an inclined opening, while provided at the side of a rear end thereof with a depressed opening, with a slit presented between the inclined opening and the depressed opening. The needle head is protruded out of the safety sheath when the needle wing is provided within the inclined opening, while the needle head is covered within the safety sheath when the needle wing is pulled back to the depressed opening from the inclined opening through the slit.

In the aforementioned transfusion needle with security, the needle wing comprises a vertical edge, which is withstood by a vertical wall of the depressed opening when the needle wing is located at the position of the depressed opening.

In the aforementioned transfusion needle with security, a rear covering body of the safety sheath is provided thereon with a non-slip portion.

In the aforementioned transfusion needle with security, the inclined opening comprises a leading angle for leading the needle wing into the slit.

In the aforementioned transfusion needle with security, the thickness of the needle wing is larger than a gap of the slit.

In the following, the present invention will be described in detail in combination with attached drawings and embodiments, but not be limited thereto.

### BRIEF DESCRIPTION OF DRAWINGS

Fig. 1 is a structural perspective view of an existing transfusion needle;
Fig. 2 is a perspective view of a main body of a transfusion needle of the present invention;
Figs. 3A to 3D are top view, elevation view, right view and left view of a safety sheath of the present invention;
Figs. 4A to 4B are a perspective view and an elevation view of the structure of a transfusion needle with security of the present invention before a needle has been covered therein;
Figs. 5A to 5B are a perspective view and an elevation view of the structure of a transfusion needle with security of the present invention after a needle has been covered therein;
Fig. 5C is a partial enlarged perspective view of the structure of transfusion needle with security of the present invention illustrated in Fig. 5A.

### DETAILED DESCRIPTION

The principle of structure and operation of the present invention will be described specifically in combination with drawings as follows.

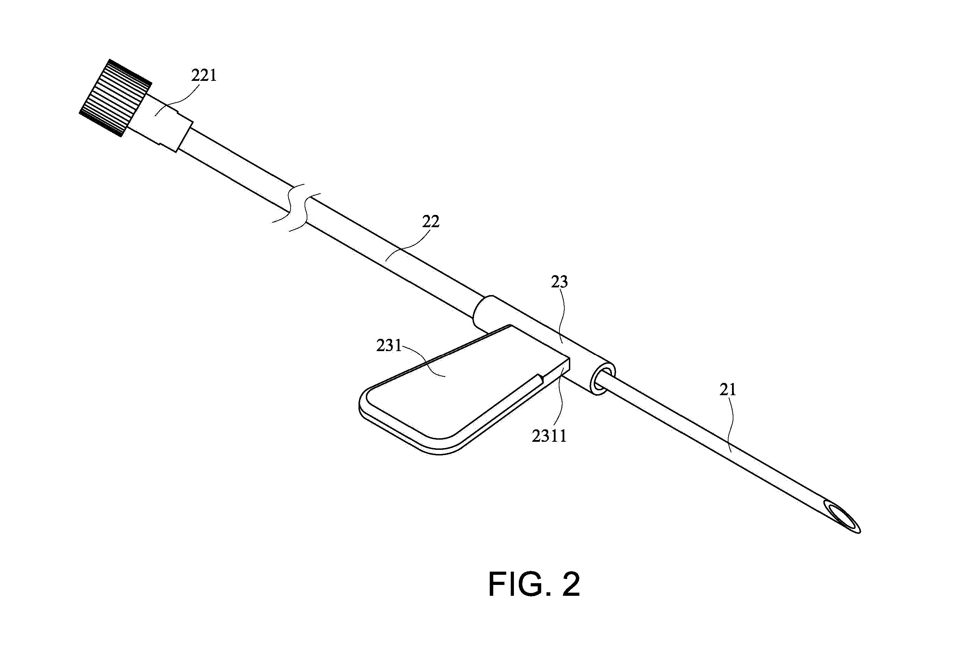

Referring to Fig. 2, there is shown a perspective view of a main body of a transfusion needle of the present invention. As illustrated in the figure, the main body 23 of the transfusion needle of the invention is fixedly provided at the side thereof with a needle wing 231, in which one end of this main body 23 is connected with a needle 21, while the other end thereof is connected with a transfusion tube 22. This transfusion tube 22 is used as a passage for medicament injection or blood flow, in which one end of this transfusion tube 22 is connected with the main body 23, while the other end thereof is provided with a joint portion 221. The joint portion 221 is used to fit with a pipe, conduit or vessel capable of containing medicament, normal saline or blood.

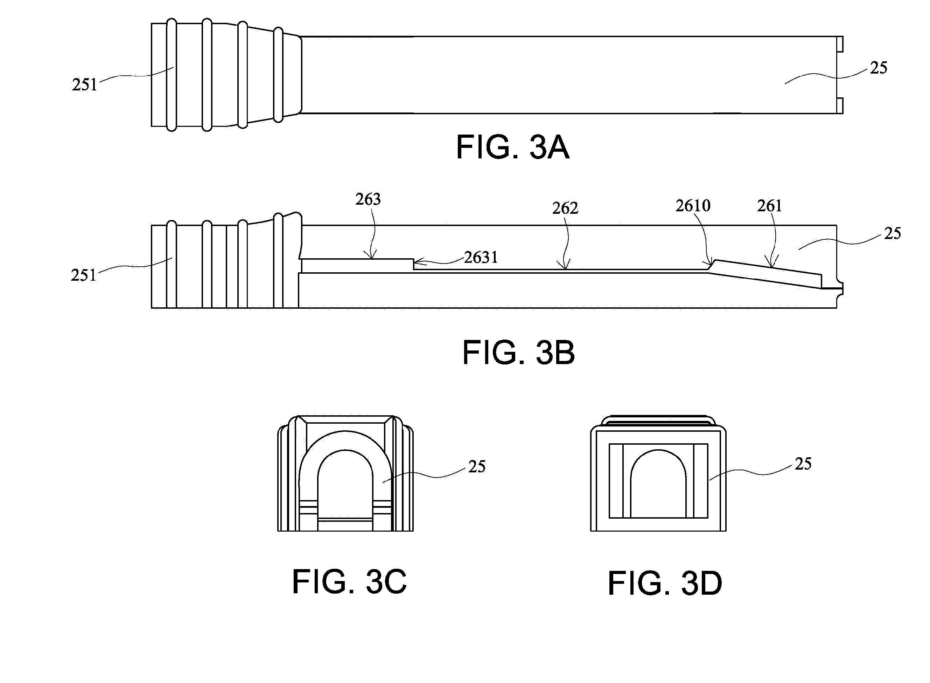

Referring to Figs. 3A to 3D, there are shown a top view, an elevation view, a right view and a left view, respectively, of a safety sheath of the present invention. As illustrated in the figures, the safety sheath 25 is a hollow covering body, which is provided at the side of the front end thereof with an inclined opening 261, while provided at the side of the rear end thereof with a depressed opening 263. Moreover, a slit 262 is presented between the inclined opening 261 and the depressed opening 263.

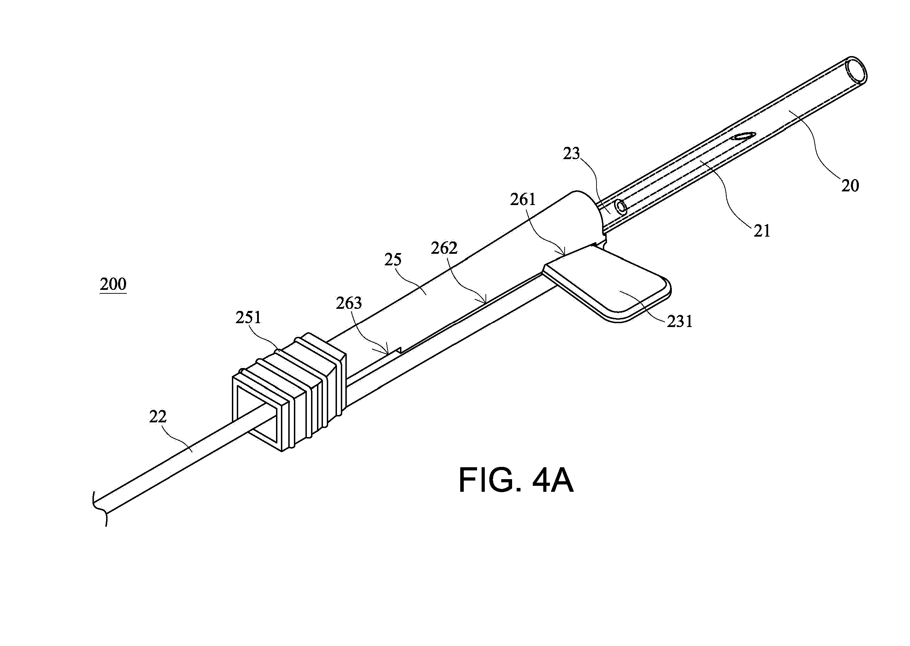

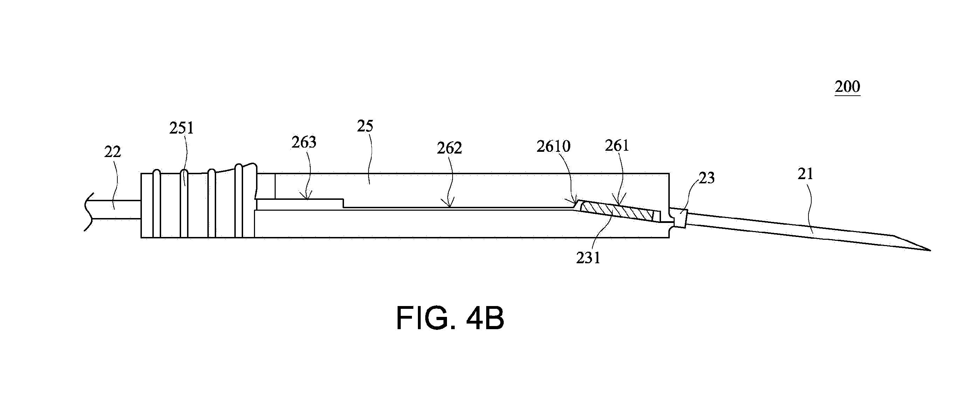

Referring to Figs. 4A to 4B, there are shown a perspective view and an elevation view, respectively, of the structure of a transfusion needle with security of the present invention before a needle has been covered therein. In the assembled structure of a transfusion needle 200 of the present invention, the main body 23 is assembled inside the safety sheath 25. Before the transfusion needle 200 is used, the needle wing 231 of the main body 23 is located at the position of inclined opening 261 of the safety sheath 25. At this moment, the needle head 21 is protruded out of the safety sheath 25 and covered with a protective needle cap 20.

When the transfusion needle 200 is used, medical personnel are allowed to remove the protective needle cap 20 covering the needle head 21, so as to expose the needle head 21 to the outside. Afterwards, the medical personnel are allowed to insert the needle head 21 into the vein, blood vessel or body of a patient via holding the needle wing 231, so as to conduct medicament injection or blood drawing. Furthermore, when the needle wing 231 is located within the inclined opening 261, the covering structure of the safety sheath 25 may be, due to incline-type design, not only pressed against the main body 23 and its needle wing 231 to form inclination, in such a way that the needle head 21 is allowed to enter into the vein of human body with a certain angle, thus avoiding hematoma resulted from pricking the vein, but also attached to the main body 23 and its needle wing 231 closely, thus avoiding slippage generated during injection.

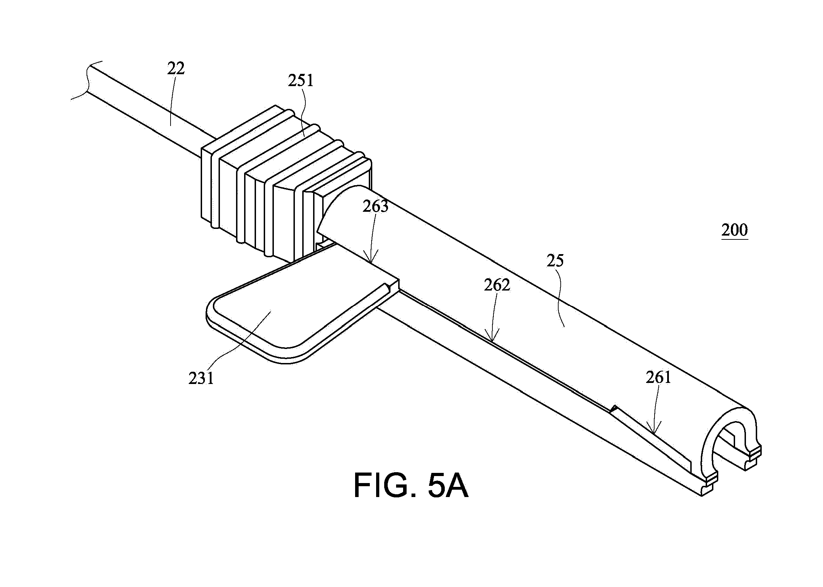

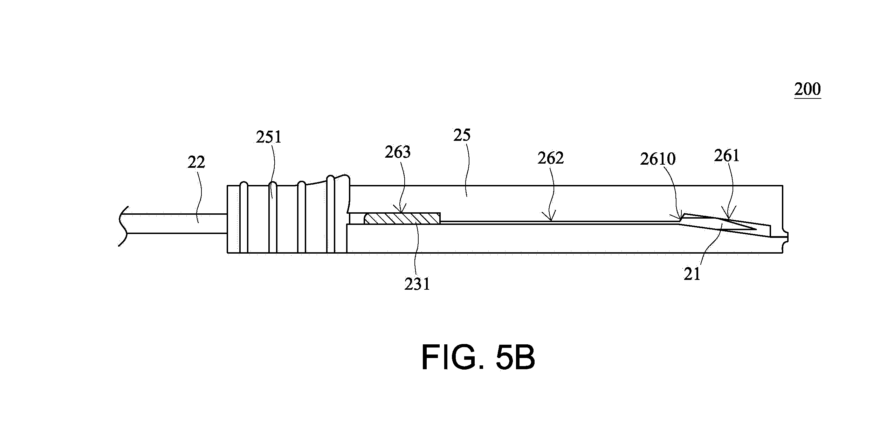

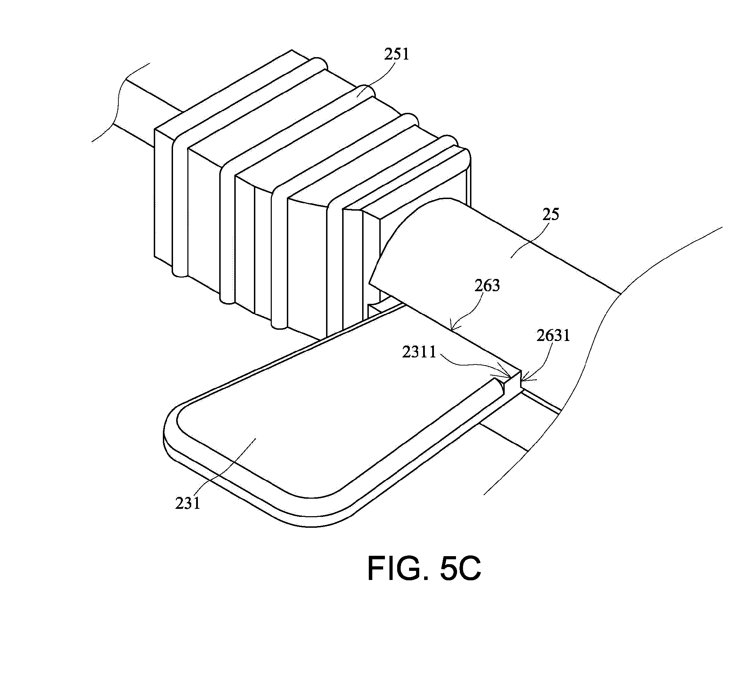

Next, referring to Figs. 5A to 5B, there are shown a perspective view and an elevation view, respectively, of the structure of a transfusion needle with security of the present invention after a needle has been covered therein. After the transfusion needle 200 has been used, medical personnel are allowed to pull out of the used needle head 21 from the body of the patient. Afterwards, when the used needle head 21 is intended to be retrieved, the medical personnel are allowed to hold the needle wing 231 and move it backwards. Then, the needle wing 231 is pulled to the position of the depressed opening 263 from the inclined opening 261 through the slit 262. At this moment, the needle head 21 is covered within the safety sheath 25 entirely. The used needle head 21 may be retrieved into the safety sheath 25 successfully and then processed as waste, due to the operation based on the above way.

In one embodiment of the present invention, the inclined opening 261 is provided with a leading angle 2610. When the needle wing 231 is pulled back from the inclined opening 261, the leading angle 2610 may be used for leading the needle wing 231 to enter into the slit 262 successfully. Moreover, in one embodiment of the present invention, the thickness of the needle wing 231 may be larger than a gap of the slit 262. When the needle wing 231 is allowed to pass through the slit 26, a part of covering body of the safety sheath 25 will be deformation generated by the slit 262 may be propped open. Afterwards, when the needle wing 231 is allowed to pass through the slit 262 and then into the depressed opening 263, the covering body of the safety sheath 25 being deformed formerly may be restored to the original form. Thus, the needle wing 231 is prevented from entering into the slit 262 again, and then locked within the depressed opening 263 firmly, due to withstanding a vertical edge 2311 of the needle wing 231 by a vertical wall 2631 of the depressed opening 263 (as illustrated in Figs. 2, 3B and 5C). Therefore, the object of retrieving the needle head 21 safely may be achieved.

Further, in one embodiment of the present invention, the safety sheath 25 is provided at the rear covering body thereof with a non-slip portion 251. When the used needle head 21 is intended to be processed, the non-slip portion 251 of the safety sheath 25 may be firstly gripped by one hand, while the needle wing 231 is held and then pulled back by the other hand of medical personnel, so as to pull the needle wing 231 into the depressed opening 263 securely, owing to non-slip effect provided by the non-slip portion 251. Thus, the fingers of medical personnel will not slip on the safety sheath 25 by the non-slip portion 251 disposed on the safety sheath 25, and therefore, the risk of accidental pricking wound caused by the needle head 21 may be avoided.

Naturally, there are still various embodiments for the present invention. It should be understood that various changes and alterations could be made to the present invention by those skilled in the art without departing from the spirit and scope of the invention, and included within the scope of the appended claims.

## Claims

1. A transfusion needle with security, comprising:
a main body, provided at the side thereof with a needle wing, and connected at one end thereof with a needle head, while connected at the other end thereof with a transfusion tube; and
a safety sheath, presented as a hollow covering body for accommodating said main body, and provided at the side of a front end thereof with an inclined opening, while provided at the side of a rear end thereof with a depressed opening, with a slit presented between said inclined opening and said depressed opening, wherein said needle head being protruded out of said safety sheath when said needle wing is provided within said inclined opening, while said needle head being covered within said safety sheath when said needle wing is pulled back to said depressed opening from said inclined opening through said slit.

2. The transfusion needle with security according to Claim 1, wherein said needle wing comprises a vertical edge, which is withstood by a vertical wall of said depressed opening when said needle wing is located at the position of said depressed opening.

3. The transfusion needle with security according to Claim 1, wherein a rear covering body of said safety sheath is provided thereon with a non-slip portion.

4. The transfusion needle with security according to Claim 1, wherein said inclined opening comprises a leading angle for leading said needle wing into said slit.

5. The transfusion needle with security according to Claim 1, wherein the thickness of said needle wing is larger than a gap of said slit.
